(19) Europäisches Patentamt
European Patent Office
Office européen des brevets

(11) Veröffentlichungsnummer: **0 020 907**
**B1**

(12) # EUROPÄISCHE PATENTSCHRIFT

(45) Veröffentlichungstag der Patentschrift: **20.10.82**

(51) Int. Cl.³: **C 07 C 103/54**, C 07 C 102/00

(21) Anmeldenummer: **80102053.8**

(22) Anmeldetag: **17.04.80**

(54) **Verfahren zur Herstellung von Betainen.**

(30) Priorität: **30.06.79 DE 2926479**

(43) Veröffentlichungstag der Anmeldung: **07.01.81 Patentblatt 81/1**

(45) Bekanntmachung des Hinweises auf die Patenterteilung. **20.10.82 Patentblatt 82/42**

(84) Benannte Vertragsstaaten: **BE FR GB IT NL**

(56) Entgegenhaltungen:
**DE-A-2 063 422**
**DE-B-1 062 392**

(73) Patentinhaber: **Th. Goldschmidt AG, Goldschmidtstrasse 100, D-4300 Essen (DE)**

(72) Erfinder: **Bade, Volkbert, Dr., Graffweg 38b, D-4300 Essen 14 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 020 907 B1

# Verfahren zur Herstellung von Betainen

Die Erfindung betrifft ein Verfahren zur Herstellung von Betainen der allgemeinen Formel

$$R^1CONH(CH_2)_xN^{\oplus}R^2R^3(CH_2)_yCOO^{\ominus} \qquad I$$

wobei $R^1$ der Alkylrest einer Fettsäure mit 6 bis 18 Kohlenstoffatomen ist,
$R^2$ und $R^3$ gleich oder verschieden sind und Alkylreste mit 1 bis 4 Kohlenstoffatomen bedeuten,
x = 2 oder 3 und
y = 1, 2 oder 3 ist, durch Quaternierung der Fettsäureamide der allgemeinen Formel

$$R^1CONH(CH_2)_xNR^2R^3 \qquad II$$

mit ω-Halogenalkylcarbonsäuren der Formel

$$X(CH_2)_yCOOH \qquad III$$

wobei X ein Halogenrest ist oder deren Salze in wässriger Lösung.

In der DE-AS-1 062 392 sind keimtötende Haarwaschmittel beschrieben, welche dadurch erhalten werden, dass man Fettsäure mit Dialkylaminoalkylamin zum Säureamid umsetzt und mit Halogencarbonsäure quaterniert. Die bei dieser Reaktion entstehenden Betaine sind oberflächenaktiv und können als Reinigungsmittel verwendet werden. Sie werden insbesondere in der Kosmetik eingesetzt, z.B. als Haarwaschmittel und weisen ausgezeichnetes Wasch- und Schaumvermögen zusammen mit keimtötender Wirkung auf.

In der DE-PS-1 172 802 ist ein Badezusatz beschrieben, der dadurch gekennzeichnet ist, dass er ausgewählte Betaine enthält. Setzt man einen derartigen Badezusatz dem Badewasser zu und lässt man den Strahl des einlaufenden Wassers zu diesem Badezusatz laufen, entsteht auf dem Badewasser ein dichter, sahniger Schaum, der auch gegen Seife stabil ist.

Diese Betaine werden für die vorgenannten Zwecke in grossem Umfang eingesetzt. Dabei zeigt es sich gelegentlich, dass Personen mit sehr empfindlicher Haut Hautreizungen aufweisen, die offenbar durch Verunreinigungen bedingt sind, die herstellungsbedingt in den Betainen enthalten sind.

Umfangreiche Untersuchungen lassen vermuten, dass für diese Hautirritationen der Rest an nicht quaternierten Fettsäureamiddialkylaminen und organisch gebundenes Chlor verantwortlich zu machen ist. Im Handel erhältliche Betaine der eingangs genannten Formel weisen einen Gehalt an freien Fettsäureamiden von 0,3 bis 3 Gew.-% auf.

Es ist bekannt, dass die Quaternierungsreaktionen mit Halogencarbonsäure auch mit einem Überschuss an Halogencarbonsäure nicht quantitativ, sondern bis zu einem theoretischen Umsatz von 90% ablaufen.

Es hat nicht an Versuchen gefehlt, das im Reaktionsgemisch enthaltene nicht quaternierte Produkt zu entfernen oder in eine nicht hautreizende Verbindung umzuwandeln. So ist beispielsweise in der DE-OS-2 063 422 ein Verfahren beschrieben, das nicht umgesetzte Fettsäureamiddialkylamin durch Zugabe von Wasserstoffperoxid in das entsprechende Aminoxid zu überführen. Man erhält dabei Gemisch aus Aminoxiden neben dem gewünschten Betain. Eine derartige Verfahrensweise ist durch die Verwendung grösserer Mengen Perhydrol aufwendig und bedarf eines zusätzlichen Verfahrensschrittes. Ein weiterer Nachteil der Überführung der Aminamide in die Aminoxidamide liegt darin, dass die Reaktion ebenfalls nicht quantitativ abläuft. Ausserdem muss sichergestellt werden, dass der Überschuss an Peroxid zerstört wird, um hierdurch bedingte Hautreizungen zu vermeiden.

Der Erfindung liegt die Aufgabe zugrunde, ein Betain der eingangs genannten Formel herzustellen, welches keine Hautirritationen mehr hervorruft und frei von nicht umgesetzten Fettsäureamiddialkylaminen und organisch gebundenem Chlor, das von nicht umgesetzter ω-Halogenalkylcarbonsäure herrührt, ist.

Überraschenderweise wurde gefunden, dass dies in einfacher Weise dadurch gelingt, dass man die Quaternierungsreaktion während des gesamten Reaktionsablaufes in alkalischer Lösung, die bei 98 °C gemessen einen pH-Wert von 7,5 bis 10,5 aufweist, durchführt.

Erfindungswesentlich ist, dass der pH-Wert der alkalischen Lösung während des gesamten Reaktionsablaufes, d.h. insbesondere auch gegen Ende der Reaktion, einen Wert von 7,5 bis 10,5, gemessen bei 98 °C aufweisen muss. Bei den üblichen Verfahren des Standes der Technik arbeitet man im wesentlichen bei neutralem pH-Wert. So ist in der DE-OS-2 063 422 ausgeführt, dass die idealen Bedingungen niedere Reaktionstemperatur und neutraler pH-Wert sind. Bei diesen Bedingungen kann aber ein Umsetzungsgrad von 90% nicht überschritten werden. Es ist zwar auch bereits bekannt, die Reaktion zunächst im alkalischen Bereich beginnen zu lassen, jedoch sinkt dann im Verlauf der Reaktion der pH-Wert ab und erreicht bzw. unterschreitet den Neutralpunkt.

Die Temperatur, bei der der pH-Wert des Reaktionsmediums gemessen wird, ist sehr wesentlich, da in dem Reaktionssystem der pH-Wert temperaturabhängig ist. Zur Festlegung des geforderten pH-Bereiches ist deshalb eine Messtemperatur von 98 °C, die der üblichen Umsetzungstemperatur entspricht, gewählt worden.

Arbeitet man innerhalb des pH-Bereiches von 7,5 bis 10,5, lässt sich nach einer Reaktionszeit von etwa 3 Stunden kein Fettsäureamiddialkylamin mehr dünnschichtchromatographisch nachweisen, wobei die Nachweisgrenze bei 0,02 Gew.-% liegt.

Besonders bevorzugt ist der pH-Wert von 8 bis 10, da ab einem pH-Wert von etwa 8 der Über-

schuss an nicht umgesetztem Alkalisalz der ω-Halogenalkylcarbonsäure zersetzt wird. Will man sicherstellen, dass in dem Umsetzungsprodukt kein organisch gebundenes Chlor mehr nachweisbar ist, empfiehlt es sich, eine Reaktionszeit von etwa 8 bis 10 Stunden einzuhalten.

Führt man die Reaktion bei pH-Werten oberhalb des Wertes von 10,5 durch, so tritt eine deutliche Zersetzung des Produktes unter Phasentrennung ein. Bei Durchführung der Reaktion unterhalb eines pH-Wertes von 7,5 verzögert sich der Reaktionsablauf zunehmend bei gleichzeitiger Verschlechterung des Umsetzungsgrades. In diesem Bereich kann der angestrebte Umsetzungsgrad unter wirtschaftlich vertretbarem Aufwand nicht mehr erreicht werden.

Der Vorteil des erfindungsgemässen Verfahrens liegt darin begründet, dass im Gegensatz zu den Reinigungsverfahren des Standes der Technik, z.B. der DE-OS-2 063 422 nicht darauf abgezielt wird, nicht umgesetztes Fettsäureamiddialkylamin in eine Verbindung zu überführen, die die Haut nicht irritiert, sondern dass man erfindungsgemäss bestrebt ist, die Reaktion quantitativ ablaufen zu lassen, ohne dass Produktverunreinigungen entstehen. Das erfindungsgemäss erhaltene Umsetzungsprodukt besteht deshalb praktisch nur aus dem gewünschten Wirkstoff und nicht aus einem Gemisch aus Wirkstoff und umgesetzter Verunreinigung.

Das nach dem erfindungsgemässen Verfahren erhaltene Betain kann nach Einstellung des gewünschten pH-Bereiches in üblicher Weise konfektioniert werden. Dabei versteht man unter Konfektionierung die Einstellung der gewünschten Konzentration, gegebenenfalls Zusatz von Farbstoffen, Parfümierungsmitteln, anderen hautpflegenden Substanzen und/oder Verdickungsmitteln.

Das erfindungsgemässe Verfahren soll anhand der folgenden Beispiele noch näher erläutert werden.

Beispiel 1

Als Fettsäure wird ein Kokosfettsäuregemisch ($C_8$ bis $C_{18}$) verwendet. 295 g N-Fettsäureamidopropyl-N,N-dimethylamin und 128 g Natriummonochloracetat werden zusammen mit 775 g Wasser in einen Dreihalskolben mit Rührer und Rückflusskühler gegeben. Die Reaktionsmischung wird langsam auf 98 °C erhitzt. Durch kontinuierliche Zugabe von 50 gewichtsprozentiger wässriger Natronlauge wird der pH-Wert auf 9 gehalten. Der Verlauf der Reaktion wird durch die Bestimmung des Restgehaltes an N-Fettsäureamidopropyl-N,N-dimethylamin mittels Dünnschichtchromatographie verfolgt.

Nach ca. 3 Stunden Reaktionszeit und einem Verbrauch von ca. 0,1 Mol NaOH ist N-Fettsäureamidopropyl-N,N-dimethylamin dünnschichtchromatographisch nicht mehr nachweisbar. Nach ca. 8 bis 10 Stunden ist auch kein organisch gebundenes Chlor mehr nachweisbar.

Beispiel 2

Analog Beispiel 1 wird ein Betain aus 284 g N-Laurylamidopropyl-N,N-dimethylamin, 128 g Natriummonochloracetat und 876 g Wasser hergestellt. Durch kontinuierliche Zugabe von 50 gewichtsprozentiger wässriger Natronlauge wird während des gesamten Reaktionsverlaufs der pH-Wert auf 7,5 gehalten. Nach ca. 5 Stunden Reaktionszeit ist N-Laurylamidopropyl-N,N-dimethylamin dünnschichtchromatographisch nicht mehr nachweisbar.

Beispiel 3

Analog Beispiel 1 wird ein Betain aus 368,5 g N-Stearylamidopropyl-N,N-dimethylamin, 128 g Natriummonochloracetat und 4468 g Wasser hergestellt. Durch kontinuierliche Zugabe von 50 gewichtsprozentiger wässriger Natronlauge wird während des gesamten Reaktionsverlaufs der pH-Wert auf 10 gehalten. Nach ca. 2 Stunden Reaktionszeit ist N-Stearylamidopropyl-N,N-dimethylamin dünnschichtchromatographisch nicht mehr nachweisbar. Organisch gebundenes Chlor ist nach 7 Stunden Reaktionszeit nicht mehr nachweisbar.

**Patentansprüche**

1. Verfahren zur Herstellung von Betainen der allgemeinen Formel

$$R^1CONH(CH_2)_xN^{\oplus}R^2R^3(CH_2)_yCOO^{\ominus},$$

wobei $R^1$ der Alkylrest einer Fettsäure mit 6 bis 18 Kohlenstoffatomen ist,

$R^2$ und $R^3$ gleich oder verschieden sind und Alkylreste mit 1 bis 4 Kohlenstoffatomen bedeuten,

x = 2 oder 3 und

y = 1, 2 oder 3 ist, durch Quaternierung der Fettsäureamide der allgemeinen Formel

$$R^1CONH(CH_2)_xNR^2R^3$$

mit ω-Halogenalkylcarbonsäuren der Formel

$$X(CH_2)_yCOOH,$$

wobei X ein Halogenrest ist oder deren Salze in wässriger Lösung, dadurch gekennzeichnet, dass man die Quaternierungsreaktion während des gesamten Reaktionsablaufes in alkalischer Lösung, die bei 98 °C gemessen einen pH-Wert von 7,5 bis 10,5 aufweist, durchführt.

2. Verfahren nach Anspruch 1, dadurch gekennzeichnet, dass man die Reaktion bei einem pH-Wert von 8 bis 10 durchführt.

**Revendications**

1. Un procédé de préparation de bétaïnes de la formule générale:

$$R^1CONH(CH_2)_xN^{\oplus}R^2R^3(CH_2)_yCOO^{\ominus} \qquad I$$

dans laquelle $R^1$ est le radical alkyle d'un acide

gras contenant 6 à 18 atomes de carbone, $R^2$ et $R^3$ sont semblables ou différents et représentent des radicaux alkyle contenant 1 à 4 atomes de carbone, x = 2 ou 3 et y = 1, 2 ou 3, par quaternisation des amides d'acide gras de la formule générale:

$R^1CONH(CH_2)_xNR^2R^3$ II

avec des acides ω-halogénalkylcarboxylique de formule:

$X(CH_2)_yCOOH$ III

X étant un radical halogène, ou avec leurs sels en solution aqueuse, caractérisé par le fait que l'on conduit la réaction de quaternisation, pendant tout le déroulement de la réaction, dans une solution alcaline qui présente un pH de 7,5 à 10,5, mesuré à 98 °C.

2. Procédé selon la revendication 1, caractérisé par le fait que l'on conduit la réaction à un pH de 8 à 10.

**Claims**

1. Process for the preparation of betaines of the general formula

$R^1CONH(CH_2)_xN^{\oplus}R^2R^3(CH_2)_yCOO^{\ominus}$

wherein $R^1$ is the alkyl radical of a fatty acid with 6 to 18 carbon atoms, $R^2$ and $R^3$ are identical or different and denote alkyl radicals with 1 to 4 carbon atoms, x is 2 or 3 and y is 1, 2 or 3, by quaternisation of the fatty acid amides of the general formula

$R^1CONH(CH_2)_xNR^2R^3$

with ω-haloalkylcarboxylic acids of the formula

$X(CH_2)_yCOOH$

wherein X is a halogen radical, or with their salts, in aqueous solution, characterised in that the quaternisation reaction, over its entire course, is effected in an alkaline solution which has a pH value of 7.5 to 10.5, measured at 98 °C.

2. Process according to Claim 1, characterised in that the reaction is carried out at a pH value of 8 to 10.